# EUROPEAN PATENT SPECIFICATION

(11) **EP 4 563 123 B1**
(45) Date of publication and mention of the grant of the patent: **19.08.2026**
(21) Application number: 23212949.4
(22) Date of filing: 29.11.2023
(51) Int. Cl.: A61F 2/38, A61B 17/16, A61B 17/17, A61F 2/46, A61F 2/30

(54) **SYSTEM COMPRISING A PATELLA TRIAL IMPLANT AND A DRILLING GUIDE TOOL**
SYSTEM MIT EINEM PATELLAVERSUCHSIMPLANTAT UND EINEM BOHRFÜHRUNGSWERKZEUG
SYSTÈME COMPRENANT UN IMPLANT D'ESSAI DE ROTULE ET UN OUTIL DE GUIDAGE DE FORAGE

(43) Date of publication of application: 04.06.2025
(73) Proprietor: Aesculap AG, 78532 Tuttlingen (DE)
(72) Inventor: Snauwaert, Eliott, 52000 Chaumont (FR)
(74) Representative: DREISS Patentanwälte PartG mbB

(56) References cited:
- EP-A1- 4 197 497
- US-A1- 2013 023 890
- US-A1- 2013 079 787
- US-A1- 2014 094 818

## Description

The invention relates to a system according to the features of the preamble of claim 1.

Patella trial implants are disclosed in EP 4 197 497 A1 and allow for simple and precise repositioning of the patella trial implant on a newly cut surface of a resected patella.

US 2014/094818 A1 discloses an orthopeadic surgical instrument.

One object of the present invention is to provide a system comprising a patella trial implant which allows for simple and precise repositioning of the patella trial implant and which facilitates the step of drilling peg holes into the resected patella.

This object is solved by the system of claim 1. Preferred embodiments are defined in the dependent claims.

According to the invention, the at least one drilling hole extends along a drilling hole axis and between a posterior receiving portion and an anterior abutment portion, wherein the receiving portion has a greater cross section than the abutment portion such that a drilling guide portion of a drilling guide tool is insertable from the posterior articular surface into the receiving portion and abutting against the abutment portion.

The abutment portion provides a stop at which an end surface of a drilling guide portion may rest during the step of drilling a peg hole. One advantage of this is that a contact of the drilling guide portion with a newly cut surface of a resected patella is avoided and that the axial position of the drilling guide portion can be defined more accurately. At the same time, the drilling guide portion is guided and accommodated within the receiving portion of the drilling hole. One advantage of this is that - at least along the receiving portion - a direct contact of a drilling tool and the patella component is avoided.

In a preferred embodiment, the receiving portion is cylindrical for simple and precise accommodation of a drilling guide portion having a circular external cross section.

In a further preferred embodiment, the abutment portion is ring-shaped for simple and precise abutment of an end surface of a sleeve-shaped drilling guide portion.

In a preferred embodiment, the patella component comprises at least two drilling holes (in particular three drilling holes) extending along respective drilling hole axes and between respective posterior receiving portions and anterior abutment portions. This facilitates the preparation of a multitude of peg holes.

In particular, at least two anterior abutment portions of at least two drilling holes have respective abutment surfaces, wherein these abutment surfaces are at equidistance to the anterior contact surface. In other words: the abutment surfaces preferably extend within the same plane which is parallel to the anterior contact surface. In this way, at least two drilling guide portions can be aligned to abut at the same plane, facilitating a subsequent drilling of peg holes.

In a preferred embodiment, the anterior contact surface comprises at least one protrusion for local engagement with a newly cut surface of a resected patella. Such a protrusion may be designed a spike which penetrates into a newly cut surface of a resected patella and provides a local form-fit connection to more stably connect the patella component to the resected patella.

In particular, the fixation member is a screw, preferably a bone screw.

In a preferred embodiment, the patella component is formed in one piece having one main body including the anterior contact surface and the posterior articular surface.

In a preferred embodiment, the fixation member comprises a tool receiving surface and a radial shoulder, the anterior portion of the fixation member being configured for fixation to a resected patella, wherein the guide slot comprises a web portion extending along the guide slot and supporting the radial shoulder, wherein the guide slot comprises an additional web portion spaced apart from and extending in parallel to the web portion, the additional web portion securing the posterior portion of the fixation member to the patella component and delimiting a tool slot for access of a fixation tool to the tool receiving surface of the fixation member. In other words: the posterior portion of the fixation member is trapped between the web portion and the additional web portion but the fixation member is still movable with respect to the guide slot. However, the tool receiving surface of the fixation member is accessible via the tool slot.

In particular, the tool slot has a width which is smaller than a width of the guide slot, thus providing a reliable protection against loss of the fixation member independent of its position along the guide slot of the patella component.

To provide an easy to produce patella component, the additional web portion may be formed in one piece with a posterior material section of the patella component which includes the posterior articular surface, wherein the web portion is provided as a separate part which is connected to the patella component.

Preferably, the separate part is connected to the patella component by means of welding, in particular by means of laser welding.

According to the invention, the drilling guide tool has a support element supporting the at least one drilling guide portion, wherein the at least one drilling guide portion has a size which allows for a form-fit engagement of the drilling guide portion with the receiving portion of the at least one drilling hole of the patella trial implant. The form-fit engagement may involve a clearance fit allowing for minimal movement of the drilling guide portion within the receiving portion. The minimal movement may be limited to e.g. 1 mm or less.

Preferably, the drilling guide portion is provided by a drilling sleeve. Such a drilling sleeve has a ring-shaped cross-section with an exterior surface to be guided within the receiving portion and with an interior surface providing guidance for a drilling tool.

In particular, the drilling guide portion has a free cross-section providing a drilling tool passage for a drilling tool, wherein the free cross-section is smaller than a free cross-section of the abutment portion of the patella trial implant. This way, a direct contact of a drilling tool and the patella component can be avoided along the entire length of the drilling hole.

Preferably the support element supports at least two drilling guide portions thus facilitating the drilling of at least two peg holes and without the need of repositioning a single drilling guide portion.

In particular, the at least two drilling guide portions are limited by respective end surfaces facing away from the support element, wherein the end surfaces extend in the same plane. In this way, at least two drilling guide portions can be aligned to abut at the same plane, facilitating a subsequent drilling of peg holes. Preferably, the end surfaces are ring-shaped.

In particular, the end surfaces which extend extend in the same plane are equidistant to a drill tool receiving surface of the support element. This way, drilling guide portions having identical guide lengths are provided, thus facilitating the drilling of peg holes having an identical drilling depth.

In particular, at least two drilling guide portions have a respective size allowing for a simultaneous form-fit engagement of the drilling guide portions with respective receiving portions of respective drilling holes of the patella trial implant. This way, the drilling of at least two peg holes may be conducted in the same position of the drilling guide tool and there is no need for repositioning a single drilling guide portion.

Further features and advantages are the subject of the following description and of the diagrammatic illustration of embodiments.

In the drawing:
- Figure 1: shows a top perspective view of one embodiment of a patella trial implant;
- Figure 2: shows a rear perspective view of the patella trial implant;
- Figure 3: shows a lateral view of an assembly of the patella trial implant being connected to but being still movable with respect to a resected patella;
- Figure 4: shows a top view of the assembly of Fig. 3;
- Figure 5: shows a top perspective view the assembly of Fig. 4 and of a fixation tool;
- Figure 6: shows a top view of the assembly of Fig. 4, patella trial implant being immovably fixed to the resected patella;
- Figure 7: shows a perspective view of a system including a drilling guide tool prior to engagement with a patella component of the patella trial implant;
- Figure 8: shows a perspective view of the system of Fig. 7, the drilling guide tool being engaged with the patella component;
- Figure 9: shows a top view of the system of Fig. 8;
- Figure 10: shows a sectional view of the system of Fig. 9 along the cutting line X - X (without fixation member);
- Figure 11: shows a perspective view of the system of Fig. 8 including a fixation tool;
- Figure 12: shows a perspective view of the system of Fig. 8 including a drilling tool prior to engagement with the drilling guide tool; and
- Figure 13: shows a perspective view of the system of Fig. 12, the drilling tool being engaged with the drilling guide tool and with the patella component and with the resected patella.

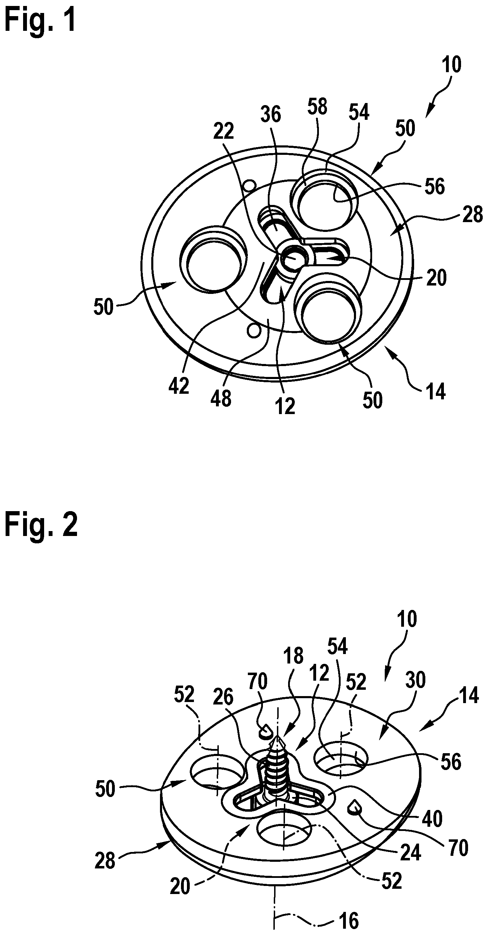

Fig. 1 and 2 show a patella trial implant 10 comprising a fixation member 12 and a patella component 14.

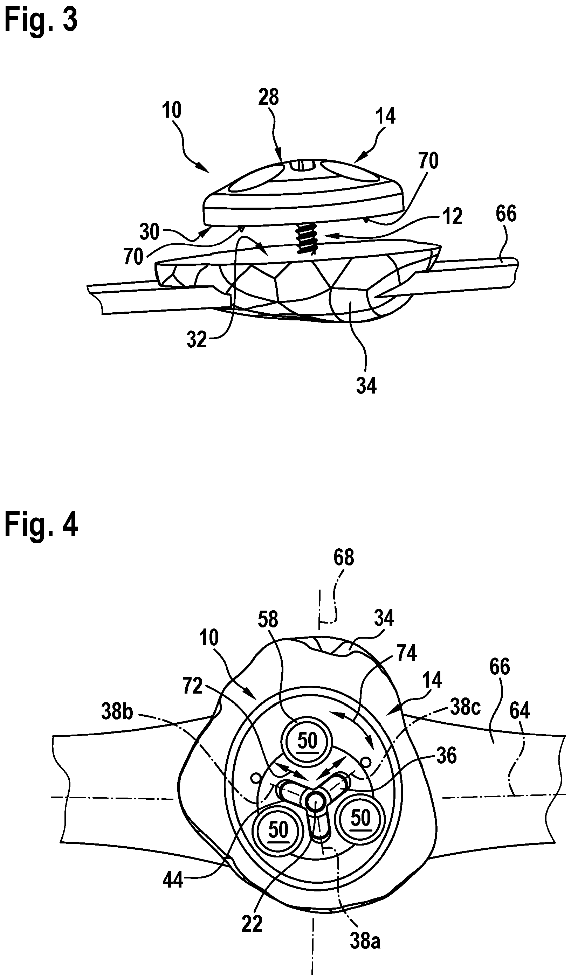

The fixation member 12 has a longitudinal axis 16 extending between an anterior portion 18 and a posterior portion 20 of the fixation member 12. The posterior portion 20 comprises a tool receiving surface 22 and a radial shoulder 24. The anterior portion 18 comprises a thread 26 which can be screwed into a resected patella (see Fig. 3).

The patella component 14 has a posterior articular surface 28 configured to articulate with a femoral surface and an anterior contact surface 30 configured to contact a newly cut surface 32 of a resected patella 34.

The patella component 14 has a guide slot 36 defining at least one guide axis 38a, 38b, 38c extending in parallel to the anterior contact surface 30 (see Fig. 4). The fixation member 12 engages in the guide slot 36, and the patella component 14 and the fixation member 12 are movable relative to each other in translation along the at least one guide axis 38a, 38b, 38c and in rotation about the longitudinal axis 16 of the fixation member 12.

The guide slot 36 comprises a web portion 40 (see Fig. 2) extending along the guide slot 36 and supporting the radial shoulder 24 of the fixation member 12. The guide slot 36 further comprises an additional web portion 42 (see Fig. 1) spaced apart from and extending in parallel to the web portion 40.

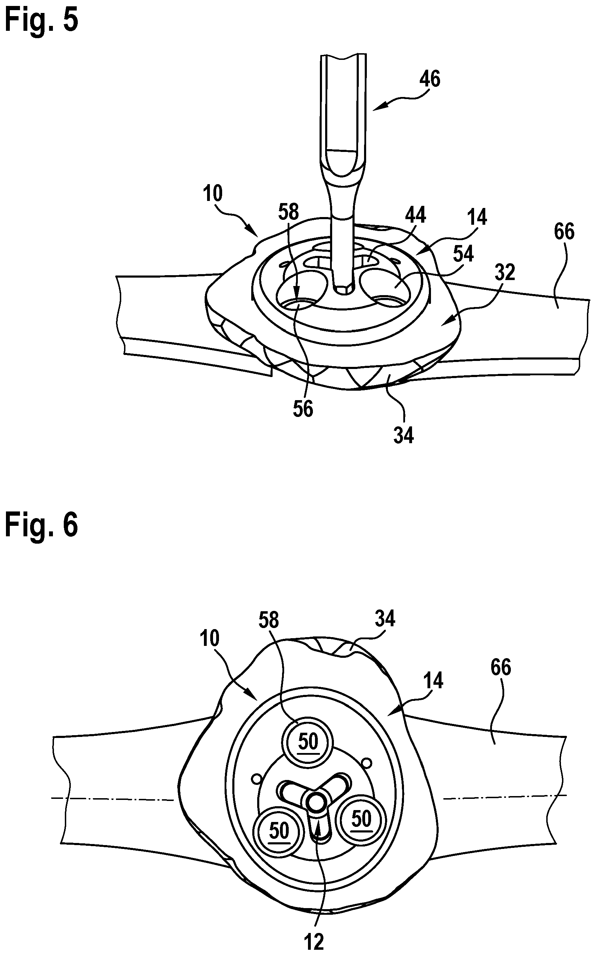

The web portion 40 and the additional web portion 42 secure the posterior portion 20 of the fixation member 12 to the patella component 14. The additional web portion 42 delimits a tool slot 44 for access of a fixation tool 46 to the tool receiving surface 22 of the fixation member 12 (see Figures 4 and 5). The width of the tool slot 44 is smaller than a width of the guide slot 36.

The additional web portion 42 is formed in one piece with a posterior material section 48 of the patella component 14 which includes the posterior articular surface 28 (see Fig. 1). The web portion 40 is provided as a separate part (see Fig. 2) which is connected to the patella component 14, in particular by laser welding.

The patella component 14 has a plurality of drilling holes 50 each extending along a drilling hole axis 52 and between a cylindrical posterior receiving portion 54 and a ring-shaped anterior abutment portion 56, see Figures 1 and 2. The anterior abutment portions 56 have respective abutment surfaces 58, wherein the abutment surfaces 58 are at equidistance to the anterior contact surface 30.

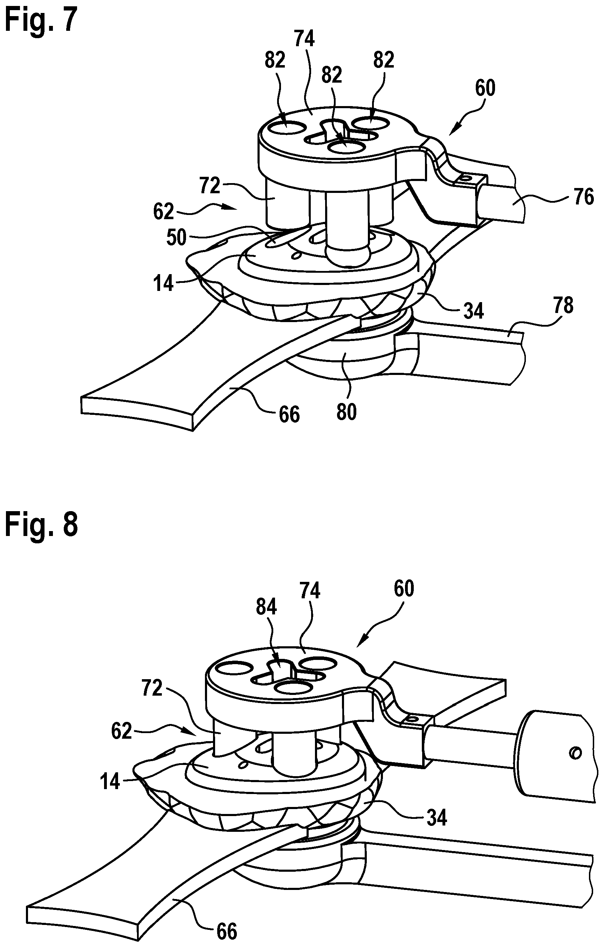

The receiving portion 54 has a greater cross section than the abutment portion 56 such that a drilling guide portion 62 of a drilling guide tool 60 (see Figures 7 and 8) is insertable from the posterior articular surface 28 of the patella component 14 into the receiving portion 54 and abutting against the abutment portion 56.

The newly cut surface 32 of the resected patella 34 extends in parallel to a proximal-distal axis 64, corresponding to an extension direction of a patellar tendon 66, and in parallel to a medio-lateral axis 68.

The patella component 14 further comprises protrusions 70 (see Figures 2 and 3) which protrude from the anterior contact surface 30 and which are to be engaged with the patella 34.

To position and secure the patella component 14 to the patella 34, the trial implant 10 is positioned such that the anterior portion 18 of the fixation member 12 contacts the newly cut surface 32 of the patella 34. Using a fixation tool 46, the fixation member 12 is threaded into the patella 34 such that the newly cut surface 32 of the patella 34 and the anterior contact surface 30 of the patella component 14 are still distant from one another (in particular such that the protrusions 70 do not yet touch the newly cut surface 32), see Fig. 3.

In a next step, the patella component 14 can be positioned in relation to the patella 34, in particular in a transitional manner along the guide axes 38a, 38b, 38c as indicated by transitional arrows 72, and in a rotational manner, as indicated by rotational arrows 74 in Fig. 4.

In a next step, the patella component 14 is secured to the patella 34 by means of screwing the anterior portion 18 of fixation member 12 into the patella 34 by means of the fixation tool 46, see Figures 5 and 6. This step involves the protrusions 70 penetrating the newly cut surface 32 and providing a protection against twisting of the patella component 14 in relation to the patella 34.

In order to prepare the drilling of peg holes into the patella 34, a system is provided which is shown in Figures 7 to 15. This system comprises a drilling guide tool 60 having drilling guide portions 62 which are provided by drilling sleeves 72 which are attached to a support element 74.

The drilling guide tool 60 comprises two arms 76 and 78, wherein a first arm 76 comprises the support element 18 and the drilling guide portions 62 to be arranged on the posterior of the patella 34 and wherein a second arm 78 comprises a counterholder 80 to to be arranged on the anterior of the patella 34.

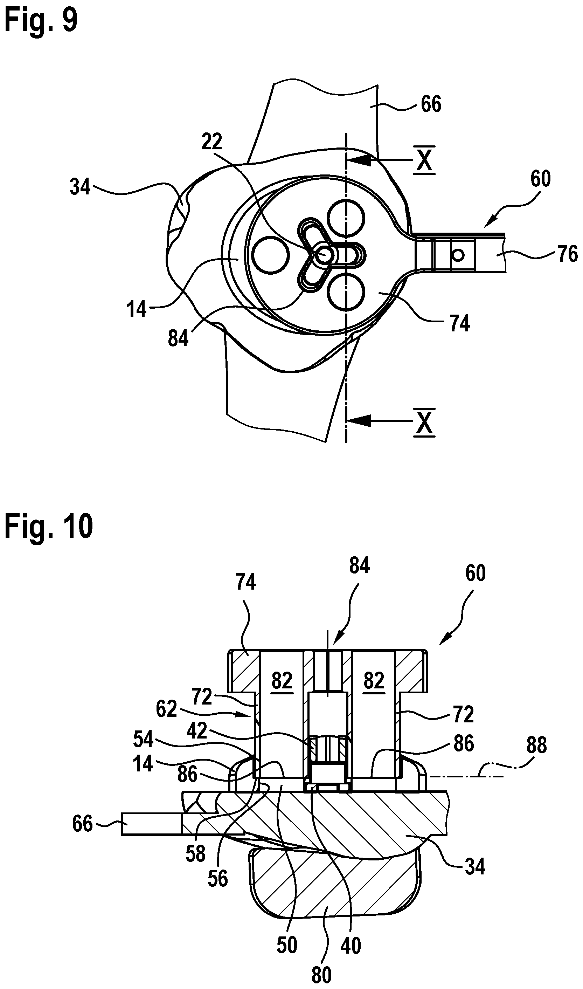

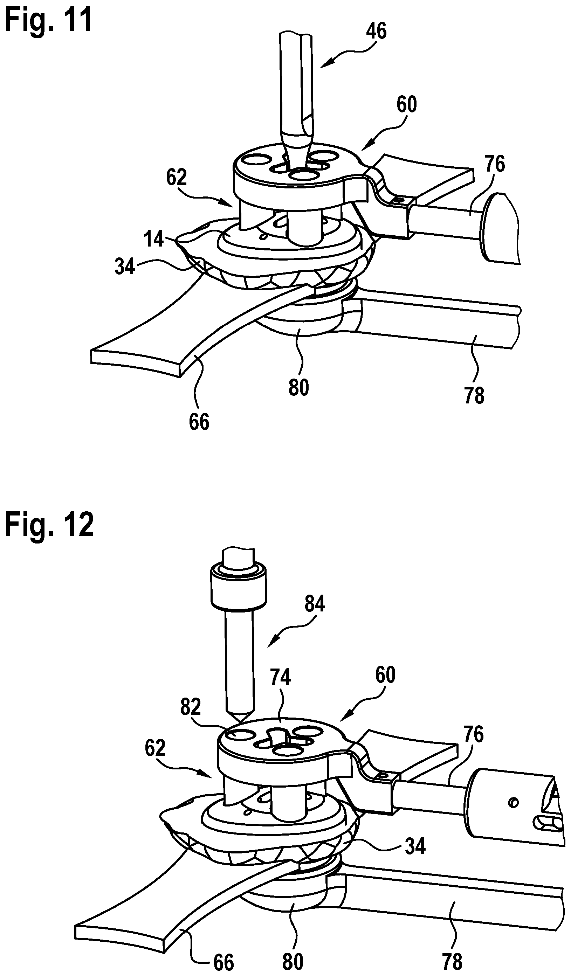

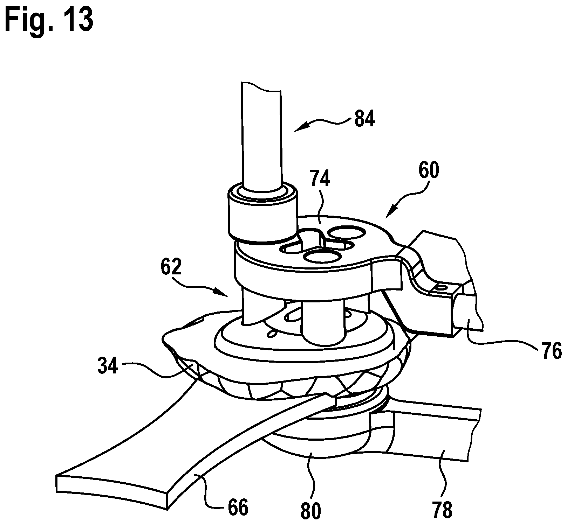

The support element 74 and the sleeves 72 provide drilling tool passages 82 (see Figures 7 and 10) for a drilling tool 84 (see Figures 12 and 13). The support element 74 further comprises a fixation tool passage 84 (see Figures 8 and 10) for the fixation tool 46 (see Fig. 11).

The drilling guide portions are limited by respective end surfaces 86 facing away from the support element 74 and preferably extending in the same plane 88, see Fig. 10.

In a first drilling preparation step, the drilling guide portions 62 of drilling guide tool 60 are inserted into the receiving portions 54 of the drilling holes 50 of the patella component 14 until the end surfaces 86 abut to the abutment surfaces 58 of the abutment portions 56, see Figures 7 to 10.

Prior to the following drilling step, the patella trial implant 10 may be repositioned with respect to the patella 34 while the drilling guide portions 62 of the drilling guide tool 60 are received within the receiving portions 54 of the patella component 14, see Fig. 11. This repositioning includes the use of the fixation tool 46 which is inserted into the fixation tool passage 84 of the support element 74, into the tool slot 44 and into the tool receiving surface 22 of the fixation member 12. The fixation member 12 may be unscrewed to an extent which allows for repositioning of the patella component 14 in a manner as described above with respect to Fig. 4 but with the drilling guide tool 60 being already engaged with the patella component 14.

The final drilling step involves the insertion of the drilling tool 84 into one of the drilling holes passage 82 of the drilling guide tool 60, through an abutment portion 56 and into the patella 34, see Figures 10, 12 and 13.

## Claims

1. A system comprising a drilling guide tool (60) and a patella trial implant (10), the patella trial implant comprising:
a fixation member (12) having a longitudinal axis (16) extending between an anterior portion (18) and a posterior portion (20);
a patella component (14) having a posterior articular surface (28) configured to articulate with a femoral surface and having an anterior contact surface (30) configured to contact a resected patella (34);
wherein the patella component (14) has a guide slot (36) defining at least one guide axis (38a, 38b, 38c) extending in parallel to the anterior contact surface (30), wherein the fixation member (12) engages in the guide slot (36), and wherein the patella component (14) and the fixation member (12) are movable relative to each other in translation along the at least one guide axis (38a, 38b, 38c) and in rotation about the longitudinal axis (16) of the fixation member (12);
wherein the patella component (14) comprises at least one drilling hole (50),
wherein the at least one drilling hole (50) extends along a drilling hole axis (52) and between a posterior receiving portion (54) and an anterior abutment portion (56), wherein the receiving portion (54) has a greater cross section than the abutment portion (56) such that a drilling guide portion (62) of the drilling guide tool (60) is insertable from the posterior articular surface (28) into the receiving portion (54) and abutting against the abutment portion (56),
**characterized in that** the drilling guide tool (60) has a support element (74) supporting the at least one drilling guide portion (62), wherein the at least one drilling guide portion (62) has a size which allows for a form-fit engagement of the drilling guide portion (62) with the receiving portion (54) of the at least one drilling hole (50) of the patella trial implant (10).

2. The system according to claim 1, **characterized in that** the receiving portion (54) is cylindrical and/or **in that** the abutment portion (56) is ring-shaped.

3. The system according to one of the preceding claims, **characterized in that** the patella component (14) comprises at least two drilling holes (50) extending along respective drilling hole axes (52) and between respective posterior receiving portions (54) and anterior abutment portions (56).

4. The system according to claim 3, **characterized in that** the anterior abutment portions (56) have respective abutment surfaces (58), wherein the abutment surfaces are at equidistance to the anterior contact surface (30).

5. The system according to one of the preceding claims, **characterized in that** the anterior contact surface (30) comprises at least one protrusion (70) for local engagement with a newly cut surface (32) of a resected patella (34).

6. The system according to one of the preceding claims, **characterized in that** the patella component (14) is formed in one piece having one main body including the anterior contact surface (30) and the posterior articular surface (32).

7. The system according to one of the preceding claims, **characterized in that** the fixation member (12) comprises a tool receiving surface (22) and a radial shoulder (24), the anterior portion (18) of the fixation member (12) being configured for fixation to a resected patella (34), wherein the guide slot (36) comprises a web portion (40) extending along the guide slot (36) and supporting the radial shoulder (24), wherein the guide slot (36) comprises an additional web portion (42) spaced apart from and extending in parallel to the web portion (40), the additional web (42) portion securing the posterior (20) portion of the fixation member (12) to the patella component (14) and delimiting a tool slot (44) for access of a fixation tool (46) to the tool receiving surface (22) of the fixation member (12).

8. The system according to claim 7, **characterized in that** the tool slot (44) has a width which is smaller than a width of the guide slot (36).

9. The system according to claim 7 or 8, **characterized in that in that** the additional web portion (42) is formed in one piece with a posterior material section (48) of the patella component (14) which includes the posterior articular surface (28) and **in that** the web portion (40) is provided as a separate part which is connected to the patella component (14).

10. The system according to claim 9, **characterized in that** the separate part is welded to the patella component (14).

11. The system according to one of the preceding claims, **characterized in that** the drilling guide portion (62) is provided by a drilling sleeve (72).

12. The system according to one of the preceding claims, **characterized in that** the drilling guide portion (62) has a free cross-section providing a drilling tool passage (82) for a drilling tool (84), wherein the free cross-section is smaller than a free cross-section of the abutment portion (56) of the patella trial implant (10).

13. The system according to one of the preceding claims, **characterized in that in that** the support element (74) supports at least two drilling guide portions (62).

14. The system according to claim 13, **characterized in that** the at least two drilling guide portions (62) are limited by respective end surfaces (86) facing away from the support element (74), the end surfaces (86) extending in the same plane.

15. The system according to claim 13 or 14, **characterized in that** the at least two drilling guide portions (62) have a respective size allowing for a simultaneous form-fit engagement of the drilling guide portions (62) with respective receiving portions (54) of respective drilling holes (50) of the patella trial implant (10).

## Patentansprüche

1. System, umfassend ein Bohrführungswerkzeug (60) und ein Patellaprobeimplantat (10), das Patellaprobeimplantat umfassend:
ein Fixierungsglied (12), das eine Längsachse (16) aufweist, die sich zwischen einem anterioren Abschnitt (18) und einem posterioren Abschnitt (20) erstreckt;
eine Patellakomponente (14), die eine posteriore artikulare Oberfläche (28) aufweist, die konfiguriert ist, um mit einer femoralen Oberfläche zu artikulieren, und eine anteriore Berührungsoberfläche (30) aufweist, die konfiguriert ist, um eine resezierte Patella (34) zu berühren;
wobei die Patellakomponente (14) einen Führungsschlitz (36) aufweist, der mindestens eine Führungsachse (38a, 38b, 38c) definiert, die sich parallel zu der anterioren Berührungsoberfläche (30) erstreckt, wobei das Fixierungsglied (12) in den Führungsschlitz (36) eingreift, und wobei die Patellakomponente (14) und das Fixierungsglied (12) translatorisch entlang der mindestens einen Führungsachse (38a, 38b, 38c) und rotatorisch um die Längsachse (16) des Fixierungsglieds (12) herum relativ zueinander bewegbar sind; wobei die Patellakomponente (14) mindestens ein Bohrloch (50) umfasst,
wobei sich das mindestens eine Bohrloch (50) entlang einer Bohrlochachse (52) und zwischen einem posterioren Aufnahmeabschnitt (54) und einem anterioren Anlageabschnitt (56) erstreckt, wobei der Aufnahmeabschnitt (54) derart einen größeren Querschnitt als der Anlageabschnitt (56) aufweist, dass ein Bohrführungsabschnitt (62) des Bohrführungswerkzeugs (60) von der posterioren artikularen Oberfläche (28) in den Aufnahmeabschnitt (54) einführbar ist und an dem Anlageabschnitt (56) anliegt,
**dadurch gekennzeichnet, dass** das Bohrführungswerkzeug (60) ein Stützelement (74) aufweist, das den mindestens einen Bohrführungsabschnitt (62) stützt, wobei der mindestens eine Bohrführungsabschnitt (62) eine Größe aufweist, die einen formschlüssigen Eingriff des Bohrführungsabschnitts (62) mit dem Aufnahmeabschnitt (54) des mindestens einen Bohrlochs (50) des Patellaprobeimplantats (10) ermöglicht.

2. System nach Anspruch 1, **dadurch gekennzeichnet, dass** der Aufnahmeabschnitt (54) zylindrisch ist und/oder dass der Anlageabschnitt (56) ringförmig ist.

3. System nach einem der vorstehenden Ansprüche,
**dadurch gekennzeichnet, dass** die Patellakomponente (14) mindestens zwei Bohrlöcher (50) umfasst, die sich entlang jeweiliger Bohrlochachsen (52) und zwischen jeweiligen posterioren Aufnahmeabschnitten (54) und anterioren Anlageabschnitten (56) erstrecken.

4. System nach Anspruch 3, **dadurch gekennzeichnet, dass** die anterioren Anlageabschnitte (56) jeweilige Anlageoberflächen (58) aufweisen, wobei die Anlageoberflächen in gleichem Abstand zu der anterioren Berührungsoberfläche (30) sind.

5. System nach einem der vorstehenden Ansprüche,
**dadurch gekennzeichnet, dass** die anteriore Berührungsoberfläche (30) mindestens einen Vorsprung (70) für einen lokalen Eingriff mit einer neu geschnittenen Oberfläche (32) einer resezierten Patella (34) umfasst.

6. System nach einem der vorstehenden Ansprüche,
**dadurch gekennzeichnet, dass** die Patellakomponente (14) einstückig ausgebildet ist und einen Grundkörper aufweist, der die anteriore Berührungsoberfläche (30) und die posteriore artikulare Oberfläche (32) umfasst.

7. System nach einem der vorstehenden Ansprüche,
**dadurch gekennzeichnet, dass** das Fixierungsglied (12) eine Werkzeugaufnahmeoberfläche (22) und eine radiale Schulter (24) umfasst, wobei der anteriore Abschnitt (18) des Fixierungsglieds (12) für die Fixierung an einer resezierten Patella (34) konfiguriert ist, wobei der Führungsschlitz (36) einen Stegabschnitt (40) umfasst, der sich entlang des Führungsschlitzes (36) erstreckt und die radiale Schulter (24) stützt, wobei der Führungsschlitz (36) einen zusätzlichen Stegabschnitt (42) umfasst, der von dem Stegabschnitt (40) beabstandet ist und sich parallel zu diesem erstreckt, wobei der zusätzliche Stegabschnitt (42) den posterioren (20) Abschnitt des Fixierungsglieds (12) an der Patellakomponente (14) sichert und einen Werkzeugschlitz (44) für den Zugang eines Fixierungswerkzeugs (46) zu der Werkzeugaufnahmeoberfläche (22) des Fixierungsglieds (12) begrenzt.

8. System nach Anspruch 7, **dadurch gekennzeichnet, dass** der Werkzeugschlitz (44) eine Breite aufweist, die kleiner als eine Breite des Führungsschlitzes (36) ist.

9. System nach Anspruch 7 oder 8, **dadurch gekennzeichnet, dass** der zusätzliche Stegabschnitt (42) einstückig mit einem posterioren Materialabschnitt (48) der Patellakomponente (14) ausgebildet ist, der die posteriore artikulare Oberfläche (28) umfasst, und dass der Stegabschnitt (40) als ein separates Teil bereitgestellt ist, das mit der Patellakomponente (14) verbunden ist.

10. System nach Anspruch 9, **dadurch gekennzeichnet, dass** das separate Teil mit der Patellakomponente (14) verschweißt ist.

11. System nach einem der vorstehenden Ansprüche,
**dadurch gekennzeichnet, dass** der Bohrführungsabschnitt (62) durch eine Bohrhülse (72) bereitgestellt wird.

12. System nach einem der vorstehenden Ansprüche,
**dadurch gekennzeichnet, dass** der Bohrführungsabschnitt (62) einen freien Querschnitt aufweist, der einen Bohrwerkzeugdurchgang (82) für ein Bohrwerkzeug (84) bereitstellt, wobei der freie Querschnitt kleiner als ein freier Querschnitt des Anlageabschnitts (56) des Patellaprobeimplantats (10) ist.

13. System nach einem der vorstehenden Ansprüche,
**dadurch gekennzeichnet, dass** das Stützelement (74) mindestens zwei Bohrführungsabschnitte (62) stützt.

14. System nach Anspruch 13, **dadurch gekennzeichnet, dass** die mindestens zwei Bohrführungsabschnitte (62) durch jeweilige Endoberflächen (86) begrenzt sind, die von dem Stützelement (74) abgewandt sind, wobei sich die Endoberflächen (86) in derselben Ebene erstrecken.

15. System nach Anspruch 13 oder 14, **dadurch gekennzeichnet, dass** die mindestens zwei Bohrführungsabschnitte (62) eine jeweilige Größe aufweisen, die einen gleichzeitigen formschlüssigen Eingriff der Bohrführungsabschnitte (62) mit jeweiligen Aufnahmeabschnitten (54) von jeweiligen Bohrlöchern (50) des Patellaprobeimplantats (10) ermöglicht.

## Revendications

1. Un système comprenant un outil guide de perçage (60) et un implant d'essai rotulien (10), l'implant d'essai rotulien comprenant :
un élément de fixation (12) ayant un axe longitudinal (16) s'étendant entre une portion antérieure (18) et une portion postérieure (20) ;
un composant rotulien (14) ayant une surface articulaire postérieure (28) configurée pour s'articuler avec une surface fémorale et ayant une surface de contact antérieure (30) configurée pour entrer en contact avec une rotule réséquée (34) ;
où le composant rotulien (14) présente une rainure de guidage (36) définissant au moins un axe de guidage (38a, 38b, 38c) s'étendant en parallèle à la surface de contact antérieure (30), dans laquelle l'élément de fixation (12) s'engage, et où le composant rotulien (14) et l'élément de fixation (12) sont mobiles l'un par rapport à l'autre en translation le long dudit ou desdits axes de guidage (38a, 38b, 38c) et en rotation autour de l'axe longitudinal (16) de l'élément de fixation (12) ; où le composant rotulien (14) comprend au moins un trou de perçage (50),
où le ou les trous de perçage (50) s'étendent le long d'un axe du trou de perçage (52) et entre une portion réceptrice postérieure (54) et une portion d'appui antérieure (56), la portion réceptrice (54) ayant une section transversale plus grande que la portion d'appui (56) de sorte qu'une douille de guidage de perçage (62) de l'outil guide de perçage (60) peut être insérée depuis la surface articulaire postérieure (28) dans la portion réceptrice (54) et venir en appui contre la portion d'appui (56),
**caractérisé en ce que** l'outil guide de perçage (60) comporte un élément de support (74) supportant au moins une douille de guidage de perçage (62), ladite douille (62) ayant des dimensions permettant un ajustement par emboîtement avec la portion réceptrice (54) du ou des trous de perçage (50) de l'implant d'essai rotulien (10).

2. Le système selon la revendication 1, **caractérisé en ce que** la portion réceptrice (54) est cylindrique et/ou **en ce que** la portion d'appui (56) est annulaire.

3. Le système selon l'une quelconque des revendications précédentes, **caractérisé en ce que** le composant rotulien (14) comporte au moins deux trous de perçage (50) s'étendant le long des axes respectifs des trous de perçage (52) et entre les portions réceptrices postérieures respectives (54) et les portions d'appui antérieures (56).

4. Le système selon la revendication 3, **caractérisé en ce que** les portions d'appui antérieures (56) présentent des surfaces d'appui respectives (58), ces surfaces d'appui étant équidistantes de la surface de contact antérieure (30).

5. Le système selon l'une quelconque des revendications précédentes, **caractérisé en ce que** la surface de contact antérieure (30) comporte au moins une protrusion (70) pour un engagement local avec une surface nouvellement coupée (32) d'une rotule réséquée (34) .

6. Le système selon l'une des revendications précédentes, **caractérisé en ce que** le composant rotulien (14) est réalisé en une seule pièce présentant un corps principal comprenant la surface de contact antérieure (30) et la surface articulaire postérieure (32).

7. Le système selon l'une des revendications précédentes, **caractérisé en ce que** l'élément de fixation (12) comprend une surface de réception de l'outil (22) et un épaulement radial (24), la portion antérieure (18) de l'élément de fixation (12) étant configurée pour fixation à une rotule réséquée (34), la rainure de guidage (36) comprenant une portion de nervure (40) s'étendant le long de la rainure de guidage (36) et supportant l'épaulement radial (24), la rainure de guidage (36) comprenant en outre une portion de nervure supplémentaire (42) espacée et parallèle à la portion de nervure (40), la portion de nervure supplémentaire (42) assurant la fixation de la portion postérieure (20) de l'élément de fixation (12) au composant rotulien (14) et délimitant une fente d'outil (44) permettant l'accès d'un outil de fixation (46) à la surface de réception de l'outil (22) de l'élément de fixation (12).

8. Le système selon la revendication 7, **caractérisé en ce que** la fente d'outil (44) a une largeur inférieure à celle de la rainure de guidage (36) .

9. Le système selon la revendication 7 ou 8, **caractérisé en ce que** la portion de nervure supplémentaire (42) est formée en une seule pièce avec une section matérielle postérieure (48) du composant rotulien (14) qui comprend la surface articulaire postérieure (28) et **en ce que** la portion de nervure (40) est fournie comme une pièce séparée qui est connectée au composant rotulien (14).

10. Le système selon la revendication 9, **caractérisé en ce que** la pièce séparée est soudée au composant rotulien (14).

11. Le système selon l'une des revendications précédentes, **caractérisé en ce que** la douille de guidage de perçage (62) est fournie par une douille de perçage (72).

12. Le système selon l'une des revendications précédentes, **caractérisé en ce que** la douille de guidage de perçage (62) présente une section transversale libre fournissant un passage pour outil de perçage (82) pour un outil de perçage (84), la section transversale libre étant plus petite que la section transversale libre de la portion d'appui (56) de l'implant d'essai rotulien (10).

13. Le système selon l'une des revendications précédentes, **caractérisé en ce que** l'élément de support (74) supporte au moins deux douilles de guidage de perçage (62).

14. Le système selon la revendication 13, **caractérisé en ce que** les au moins deux douilles de guidage de perçage (62) sont limitées par des surfaces d'extrémité respectives (86) orientées à l'opposé de l'élément de support (74), les surfaces d'extrémité (86) s'étendant dans un même plan.

15. Le système selon la revendication 13 ou 14, **caractérisé en ce que** les au moins deux douilles de guidage de perçage (62) ont une taille respective permettant un engagement par emboîtement simultané des douilles de guidage de perçage (62) avec les portions réceptrices respectives (54) des trous de perçage (50) de l'implant d'essai rotulien (10).
